# EUROPEAN PATENT APPLICATION

(11) **EP 4 035 913 A2**
(43) Date of publication of application: **03.08.2022**
(21) Application number: 22460002.3
(22) Date of filing: 11.01.2022
(51) Int. Cl.: B60H 1/00, B60H 3/00, B60H 3/02, B60H 3/06, A61L 9/00, B64D 13/06

(54) **INSTALLATION FOR LIMITING A VIRAL CROSS CONTAMINATION OF PERSONS IN A VEHICLE INTENDED FOR PUBLIC USE, AND A FILTERING DEVICE TREATING BREATHABLE AIR FOR SAID INSTALLATION**

(30) Priority: 12.01.2021 PL 43664821
(71) Applicant: Fret, Robert, 05-070 Sulejówek (PL)
(72) Inventor: Fret, Robert, 05-070 Sulejówek (PL)
(74) Representative: Fietko-Basa, Sylwia

(57) **Abstract**

Installation for limiting viral cross contamination of persons in a vehicle intended for public use, containing at least a filtering device (1) treating breathable air, adapted to draw and filter air from the interior of the vehicle or from the outside of the vehicle, connected to a treated air distribution system (2) individually for each person in the vehicle, wherein the treated air distribution system (2) consists of at least one main air trunk line (2.1) which via distributive tees (2.3) or distributive crosses (2.4) is connected to lateral air trunk lines (2.2), and distributive tees (2.5) on the lateral trunk lines (2.2) and distributive tees (2.10) and distributive crosses (2.11) on the at least one main trunk line (2.1) are connected to air connection nozzles (2.8) via adjustable valves (2.6).

## Description

An installation limiting viral cross contamination of persons in a vehicle intended for public use constituting a separate respiratory air installation protecting the persons in such a vehicle against viral cross contamination due to breathing is the subject of the invention. A filtering device treating breathable air for such an installation is also the subject of the invention. Public transport vehicles, including commercial vehicles for carrying persons are understood as vehicles intended for public use, i.e.: long-distance trains, suburban trains, trams, coaches, intercity buses, city buses, taxis, emergency ambulances, company vehicles, passenger airplanes, passenger ships, passenger ferries, elevators, etc.

Various kinds of ventilation and air-conditioning systems are already known in the prior art and used to remove polluted air from the vehicle intended for public use and replace it with fresh air. Such systems have to be applied and used because the air in the volume of the vehicle travelling on the roads gets constantly polluted, whereas, such air may be polluted (contaminated) with:
- naturally occurring atmospheric pollutants (dirt, dust, smog, microorganisms);
- naturally occurring microbiological contaminants (mites, fungal and mold spores, epidermis, animal hair, bacteria);
- pollutants of the exhaled carbon dioxide (CO2);
- pollutants discharged with exhaust gases: carbon monoxide (CO), nitrogen oxides (NOx), hydrocarbons (CH) and soot;
- bacterial and viral contaminants distributed via the air-conditioning device;
- viral contaminants in the exhaled breath of the passengers travelling in these vehicles.

The particles of the naturally occurring microbiological contaminants mentioned above do not exceed 1 micron and constitute 90% of all particles suspended in the air.

A person (driver, passenger) inside a vehicle intended for public use exhales about 500 litres of air per hour (ca. 0.5 m³/h), maximum up to 2m³/h. While the human organism as far as possible copes well with the naturally occurring contaminants, the mentioned viral contaminants in the exhaled breath of persons inside the vehicle, often additionally spread by the air-conditioning system, is a matter of concern. The filtroventilation and air conditioning systems that have normally been functioning until now in a vehicle intended for public use do not namely protect against viral infections originating in the same vehicle. People inside such public transport vehicles have to use individual protection measures against contaminations (e.g. protective masks).

Various ventilation and air-conditioning systems are known in the prior art. One of the basic conditions for carrying passengers inside a vehicle is access to fresh air secured by an adequate ventilation system.

Ventilation which is responsible for the exchange of air in a vehicle often cooperates with the air-conditioning system. The basic function of air-conditioning is air temperature adjustment inside a vehicle in order to ensure optimum conditions for travelling and breathing, irrespective of the season of the year. Most frequently air conditioning is used to cool the interior of a vehicle in summer and to heat it in the colder seasons. The advanced solutions for ventilation and air conditioning systems in higher quality vehicles cannot only cool/heat air, but also moisturize and purify it thanks to filters trapping dust, smoke, bacteria, viruses, fungi, animal hair and odors.

The issue, however, is to protect the passengers inside the vehicle against viral cross contamination.

The aim of the invention is to work out an installation limiting viral cross contamination of passengers inside a vehicle intended for public use. The aim of the invention is also to work out a filtering device for the treatment of breathable air which is appropriated for such an installation.

The installation limiting viral cross contamination of passengers in a vehicle intended for public use, according to the invention, is characterized in that it contains at least a filtering device treating breathable air adapted for drawing and filtering the air from the interior or the exterior of the vehicle which is connected to a system distributing treated air, individually for each person travelling in a vehicle, whereas, the system distributing treated air consists of at least one main air trunk line, which via distributive tees or distributive crosses is connected to the lateral air trunk lines, whereas, the distributive tees on the lateral trunk lines and the distributive tees and distributive crosses on the main trunk lines are connected to air connection nozzles via adjustable valves. Preferably, at least one air inlet valve operated manually or via an actuator is installed on one inlet to the filtering device treating breathable air. Preferably, connection nozzles of the system distributing treated air have built-in air glands.

The filtering device treating breathable air intended for the installation limiting viral cross contamination of passengers in a vehicle intended for public use according to the invention is characterized in that it consists of at least one air inlet and treated air outlets and a control panel, whereas, inside the casing there is a set of filters consisting of at least an air filter for the initial air purification, a blower, an antivirus air filter and a specialist air filter containing an activated charcoal absorber and moreover the device is preferably equipped with agents for additional treatment of air. Preferably, the filtering device contains ultra-violet (UV-C) flow lamps as agents additionally treating air. Preferably, a nozzle supplying a moisturizing agent appropriated for the inhalation of air, functioning as an agent for additional treatment of air, is inserted into the interior of the casing of the filtering device treating breathable air.

Preferably, a nozzle supplying oxygen is inserted into the interior of the casing of the filtering device as an agent for additional treatment of air. Preferably, in the interior of the casing of the filtering device treating breathable air there is a sensor detecting pressure of the treated air installed at the end of the draft where air is treated. Preferably, in the interior of the casing of the filtering device there is a sensor detecting the carbon monoxide content installed at the end of the draft where air is treated. Preferably, in the interior of the casing of the filtering device there is a sensor detecting the oxygen content in the treated air. Preferably, in the interior of the casing of the filtering device there are heat exchangers for heating or cooling treated air. Preferably, in the interior of the casing of the filtering device there is a sensor detecting the temperature of the treated air.

The use of the installation limiting viral cross contamination of persons in a vehicle intended for public use and the filtering device treating breathable air according to the invention shall reduce the risk of viral cross contamination caused by passengers breathing in the vehicle, especially in cases when such persons are sitting, standing in close proximity to one another. The above installation shall allow public transport vehicles to be used in a more efficient way without the need for maintaining social distancing.

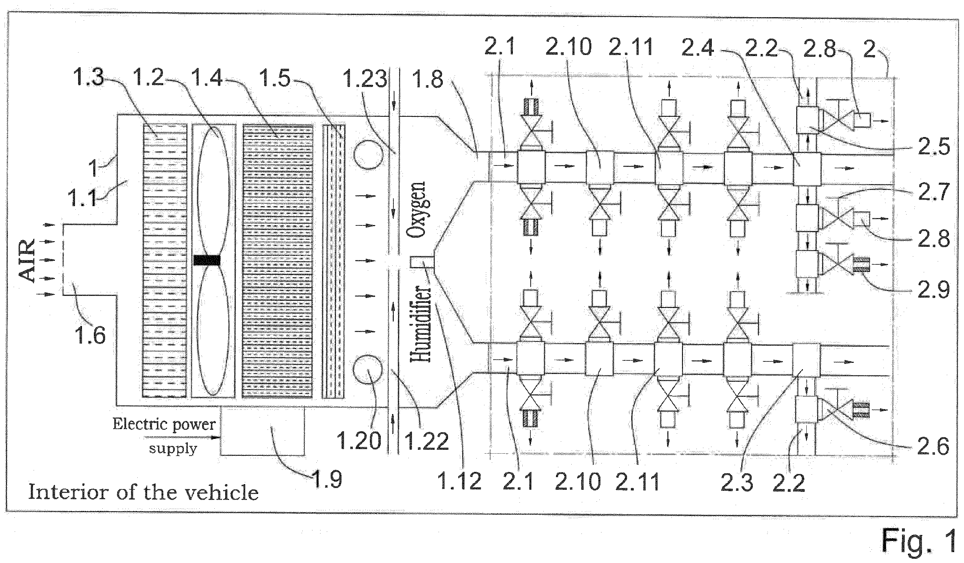

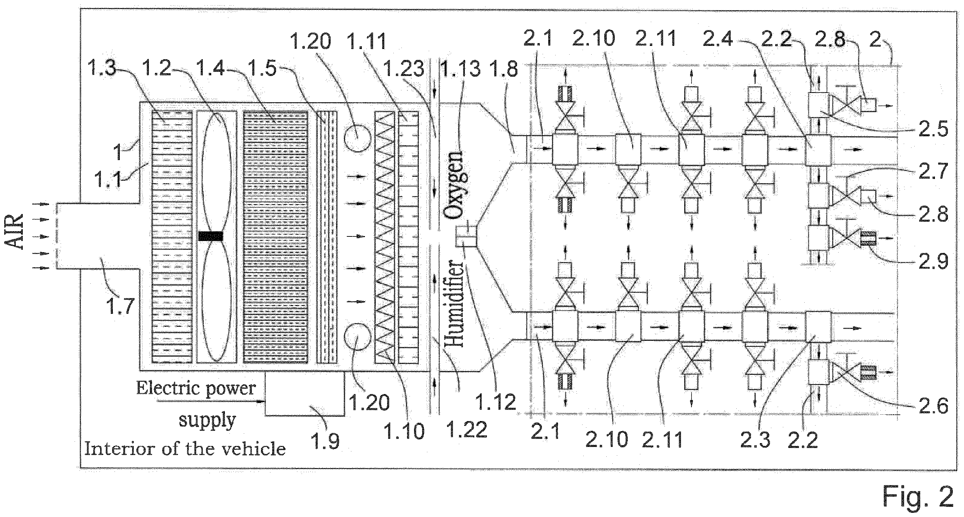

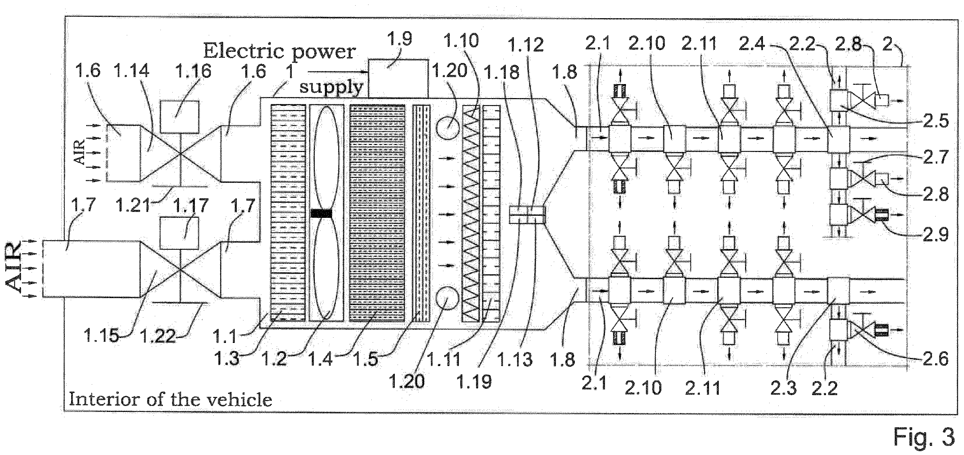

The subject of the invention is presented in embodiments in the drawing where figure 1 presents the installation limiting viral cross contamination of passengers in a vehicle intended for public use taking air in from the interior of the vehicle and the filtering device treating breathable air which is a part of this installation in the first embodiment, figure 2 presents the same installation in the second embodiment where air is drawn from the environment outside the vehicle and treated in a range necessary for breathing and distributed individually for breathing for each person, whereas, the device additionally contains an exchanger system of warm and cold air, while figure 3 presents the installation in the third embodiment where air is drawn directly from the interior of the vehicle, as well as, from the environment outside the vehicle and treated in a range necessary for breathing and distributed individually for breathing for each person.

In the first embodiment shown in figure 1 the installation limiting viral cross contamination of passengers in a vehicle intended for public use consists of a filtering device 1 drawing air directly from the interior of the vehicle and treating the incoming air indispensable for breathing and consists of a system distributing the treated air 2 individually for each person. The treated air distribution system 2 consists of two main air trunk lines 2.1 and distributive tees 2.3 and distributive crosses 2.4 redistributing the treated air into respective lateral trunk lines 2.2.

Distributive tees 2.10 and distributive crosses 2.11 are directly connected to the main trunk lines 2.1 and tees 2.5 taking air out to the valves 2.6 manually operated by handwheels 2.7 with connection nozzles 2.8 inside of which glands 2.9 are built in limiting the maximum air volume per person are directly connected to lateral trunk lines. The system distributing treated air 2 provides treated breathable air individually for each person, after being connected to the connection nozzle 2.8 by means of additional equipment already known in the prior art and not shown in the drawing.

The filtering device 1 treating breathable air consists of a casing 1.1 equipped with one air inlet 1.6 placed inside the vehicle and taking air in from its interior and two treated air outlets 1.8 and also a control panel 1.9. Inside the casing 1.1 there is a set of filters consisting of at least an air filter for initial purification 1.3 (minimum class F5), a blower 1.2, an antivirus air filter 1.4 and a specialist air filter 1.5 containing an absorber with activated charcoal and air treatment agents. Air is taken in through the initial air purifying filter 1.3 and the blower 1.2 directly from the vehicle via the air intake nozzle 1.6, then air undergoes antiviral treatment via a fine filter (a highly efficient HEPA filter minimum class H14) 1.4 and qualitative treatment via a specialist filter (absorber with activated charcoal) 1.5, whereupon it is additionally treated by agents 1.20 in the form of ultraviolet UV-C flow lamps which additionally decontaminate air. The treated air is then moved to the system distributing treated air 2 via air outlet nozzles 1.8. Moreover, the air outlet from the filtering device 1 has a built-in overpressure sensor 1.12 determining the outlet air overpressure in [Pa], whereas, air overpressure in the air outlet nozzles 1.8 is maintained within the limits indispensable for breathing due to the volume change (change in revolutions) of the blower 1.2 moving the air. Depending on the need, the outlet air from the filtering device 1 may additionally be treated via agent 1.22 in the form of a nozzle inserted into the interior of the casing 1.1 through which an inhalation agent is administered (e.g. saline solution) or via agent 1.23 in the form of a nozzle inserted into the interior of the casing 1.1 through which oxygen is administered.

In the second embodiment shown in figure 2 there is an installation limiting viral cross contamination of passengers in the vehicle intended for public use as in the first embodiment, whereas, the air inlet is situated on the outside of the vehicle. In this embodiment the filtering device 1 is additionally equipped with heat exchangers 1.10 for heating air and 1.11 for cooling air. Air temperature is controlled by a temperature sensor 1.13 situated at the outlet from the filtering device of the treated air. The heat exchangers 1.10 and 1.11 are situated behind the ultraviolet lamps 1.20

In the third embodiment shown in figure 3 the installation as in the first embodiment is adapted to take air in from the interior of the vehicle and as in the second embodiment is adapted to take air in from the environment on the outside of the vehicle. The portion of the air taken directly from the interior of the vehicle by the air intake nozzle 1.6 and air taken from the environment on the outside of the vehicle via the air intake nozzle 1.7 is controlled by the degree by which the air valves open, i.e. valve 1.14 on nozzle 1.6 and and valve 1.15 on nozzle 1.7 respectively, whereas, the degree by which the air valves 1.14 and 1.15 open is determined manually by handwheels 1.21 and 1.22 respectively or by electric actuators 1.16 and 1.17 respectively. The filtering device 1 treating breathable air is additionally equipped with an additional air content sensor 1.18 at the air outlet - determining the oxygen content in the treated exhaust air, whereas, the oxygen content in the exhaust air is controlled within the limits indispensable for breathing as above by the degree by which the air valve opens, i.e. valve 1.15 on nozzle 1.7 taking air in from the environment on the outside of the vehicle and valve 1.14 on nozzle 1.6 taking air in from the interior of the vehicle respectively.

In each embodiment an additional air content sensor 1.19 determining the occurrence of a toxic gas (carbon monoxide CO) in the treated outlet air can be installed at the air outlet from the filtering device 1 treating breathable air, whereas, the occurrence of carbon monoxide in air is signalled visually and acoustically on the control panel 1.9.

Electric supply, control and monitoring of the equipment of the filtering device 1 treating breathable air, including: the blower 1.2, heat exchangers for heating 1.10 and for cooling 1.11, ultraviolet UV-C flow lamps 1.20, electric actuators for valves 1.16 and 1.17, the over-pressure sensor 1.12, the temperature sensor 1.13, the oxygen sensor 1.18 and the CO sensor 1.19 is carried out by the control panel 1.9 powered from an external power grid.

Components comprising the filtering device 1 treating the supplied air indispensable for breathing may not be enclosed in a common casing 1.1 but freely detached in separate casings tightly interconnected with each other.

In each embodiment the system distributing treated air 2 supplies treated breathable air individually for the driver and the passengers after being connected to the connection nozzle 2.8 via additional equipment not shown in the drawing. The number of connectors 2.8 for taking treated air in by respective persons is limited by the technical parameters of the installation limiting viral cross contamination of persons inside a vehicle intended for public use, including the efficiency of the filtering device 1 in the range of capacity [m3/h] and the obtained compression [Pa].

## Claims

1. The installation limiting viral cross contamination of persons travelling in a vehicle intended for public use **is characterized in that** it contains at least a filtering device (1) treating breathable air, adapted to draw, filter and treat air coming from the interior of the vehicle or from the outside of the vehicle connected to the treated air distribution system (2) individually for each person in the vehicle, whereas, the system distributing treated air (2) consists of at least one main air trunk line (2.1) which via distributive tees (2.3) or distributive crosses (2.4) is connected to the lateral air trunk lines (2.2), whereas, the distributive tees (2.5) on the lateral trunk lines (2.2) and distributive tees (2.10) and distributive crosses (2.11) on the main trunk lines (2.1) are connected to connection nozzles for drawing treated air (2.8) individually by respective persons via adjustable valves (2.6).

2. The installation according to claim 1 is **characterized in that** the air intake valve operated manually or via an actuator (1.14), (1.15) is installed at least on one inlet to the filtering device (1).

3. The installation, according to claim 1 is **characterized in that** the connection nozzles for drawing treated air (2.8) in the system distributing treated air (2) have built-in air glands (2.9).

4. The filtering device (1) for the installation according to claims 1-3 of this invention is **characterized in that** it contains at least a casing (1.1) equipped with at least one air inlet (1.6) or (1.7) and treated air outlets (1.8) and the control panel (1.9), whereas, inside the casing (1.1) there is a set of filters consisting of at least an air filter for the initial purification (1.3), a blower (1.2), an antivirus air filter (1.4) and a specialist air filter (1.5) containing an absorber with activated charcoal and moreover the device is equipped with at least one agent allowing additional air treatment (1.20) or (1.22) or (1.23).

5. The filtering device (1) according to claim 4 is **characterized in that** it contains ultraviolet UV-C flow lamps as an agent additionally treating air (1.20).

6. The filtering device (1) according to claim 4 is **characterized in that** a nozzle supplying a moisturizing agent appropriated for the inhalation of air as an agent (1.22) additionally treating air is inserted into the interior of the casing (1.1).

7. The filtering device (1) according to claim 4 is **characterized in that** a nozzle supplying oxygen as an agent (1.23) additionally treating air is inserted into the interior of the casing (1.1).

8. The filtering device (1) according to claim 4 is **characterized in that** inside the casing (1.1) there is a sensor detecting the pressure (1.12) of treated air installed at the end of the draft where air is treated.

9. The filtering device (1) according to claim 4 is **characterized in that** inside the casing (1.1) there is a sensor detecting the carbon monoxide content (1.13) installed at the end of the draft where air is treated.

10. The filtering device (1) according to claim 4 is **characterized in that** inside the casing (1.1) there is a sensor detecting the oxygen content (1.18) in the treated air.

11. The filtering device (1) according to claim 4 is **characterized in that** it contains a heat exchanger (1.10), (1.11) for heating or cooling the treated air.

12. The filtering device (1) according to claim 4 and 11 is **characterized in that** inside the casing (1.1) there is a sensor detecting the temperature (1.19) of the treated air.
